(19) Europäisches Patentamt
European Patent Office
Office européen des brevets

(11) **EP 3 146 354 B1**

(12) # EUROPÄISCHE PATENTSCHRIFT

(45) Veröffentlichungstag und Bekanntmachung des Hinweises auf die Patenterteilung:
**12.08.2020 Patentblatt 2020/33**

(21) Anmeldenummer: **15726557.0**

(22) Anmeldetag: **19.05.2015**

(51) Int Cl.:
*G01R 33/56* (2006.01)     *G01R 33/48* (2006.01)
*G01R 33/563* (2006.01)    *G01R 33/565* (2006.01)
*A61B 5/055* (2006.01)

(86) Internationale Anmeldenummer:
**PCT/EP2015/060956**

(87) Internationale Veröffentlichungsnummer:
**WO 2015/177117 (26.11.2015 Gazette 2015/47)**

(54) **VERFAHREN ZUR ERZEUGUNG MAGNETRESONANZTOMOGRAPHISCHER AUFNAHMEN VON ZYKLISCHER BEWEGUNG**

METHOD FOR PRODUCING MAGNET RESONANCE TOMOGRAPHY RECORDINGS OF CYCLIC MOVEMENT

PROCÉDÉ D'IMAGERIE DE MOUVEMENT CYCLIQUE PAR TOMOGRAPHIE À RÉSONANCE MAGNÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.05.2014 DE 102014209437**

(43) Veröffentlichungstag der Anmeldung:
**29.03.2017 Patentblatt 2017/13**

(73) Patentinhaber: **Sirona Dental Systems GmbH**
**64625 Bensheim (DE)**

(72) Erfinder:
• **RASCHE, Volker**
 **89155 Erbach (DE)**
• **WUNDRAK, Stefan**
 **64625 Gronau (DE)**

(74) Vertreter: **Özer, Alpdeniz et al**
**Dentsply Sirona**
**Legal IP Department**
**Fabrikstraße 31**
**64625 Bensheim (DE)**

(56) Entgegenhaltungen:
• **A. D. SCOTT ET AL.: "Adaptive Averaging Applied to Dynamic Imaging of the Soft Palate", MAGNETIC RESONANCE IN MEDICINE, Bd. 70, September 2013 (2013-09), Seiten 865-874, XP002742189, in der Anmeldung erwähnt**
• **SEITZ S M ET AL: "VIEW-INVARIANT ANALYSIS OF CYCLIC MOTION", INTERNATIONAL JOURNAL OF COMPUTER VISION, KLUWER ACADEMIC PUBLISHERS, NORWELL, US, Bd. 25, Nr. 3, 1. Dezember 1997 (1997-12-01), Seiten 231-251, XP000725953, ISSN: 0920-5691, DOI: 10.1023/A:1007928103394**
• **CUTLER R ET AL: "ROBUST REAL-TIME PERIODIC MOTION DETECTION, ANALYSIS, AND APPLICATIONS", IEEE TRANSACTIONS ON PATTERN ANALYSIS AND MACHINE INTELLIGENCE, IEEE COMPUTER SOCIETY, USA, Bd. 22, Nr. 8, 1. August 2000 (2000-08-01) , Seiten 781-796, XP000976486, ISSN: 0162-8828, DOI: 10.1109/34.868681**
• **S. ZHANG ET AL.: "Real-Time Magnetic Resonance Imaging of Temporomandibular Joint Dynamics", THE OPEN MEDICAL IMAGING JOURNAL, Bd. 5, 2011, Seiten 1-7, XP002742190, in der Anmeldung erwähnt**
• **STEFANIE WINKELMANN ET AL: "An Optimal Radial Profile Order Based on the Golden Ratio for Time-Resolved MRI", IEEE TRANSACTIONS ON MEDICAL IMAGING, IEEE SERVICE CENTER, PISCATAWAY, NJ, US, Bd. 26, Nr. 1, 1. Januar 2007 (2007-01-01) , Seiten 68-76, XP011152669, ISSN: 0278-0062, DOI: 10.1109/TMI.2006.885337**

EP 3 146 354 B1

    **(Forts. nächste Seite)**

- HARDY C T ET AL: "Coronary angiography by real-time MRI with adaptive averaging", MAGNETIC RESONANCE IN MEDICINE WILEY USA, Bd. 44, Nr. 6, Dezember 2000 (2000-12), Seiten 940-946, XP000970106, ISSN: 0740-3194
- HARDY C J ET AL: "Coronary MR angiography: Respiratory motion correction with BACSPIN", JOURNAL OF MAGNETIC RESONANCE IMAGING, SOCIETY FOR MAGNETIC RESONANCE IMAGING, OAK BROOK, IL, US, Bd. 17, 22. Januar 2003 (2003-01-22), Seiten 170-176, XP002288238, ISSN: 1053-1807, DOI: 10.1002/JMRI.10250
- SHUO ZHANG ET AL: "Magnetic resonance imaging in real time: Advances using radial FLASH", JOURNAL OF MAGNETIC RESONANCE IMAGING JOHN WILEY & SONS LTD. UK, Bd. 31, Nr. 1, Januar 2010 (2010-01), Seiten 101-109, XP002601165, ISSN: 1053-1807

**Beschreibung**

Technisches Gebiet

Die Erfindung betrifft ein Verfahren zur Erzeugung magnetresonanztomographischer Aufnahmen von mindestens einer Phase einer zyklischen Bewegung.

Stand der Technik

Um magnetresonanztomographische Bilder von verschiedenen Phasen einer Bewegung eines Objekts, beispielsweise von der Kaubewegung eines Kiefers, zu erzeugen, werden häufig Aufnahmen von Einzelpositionen der Bewegung gemacht. Hierzu ist eine Vorrichtung zur Fixierung des Objekts, beispielsweise des Kiefers, in den verschiedenen Positionen notwendig. Durch die Fixierung wird es möglich, zu jeder Einzelposition ausreichend magnetresonanztomographische Daten für ein Bild zu erzeugen. Allerdings ist diese Art der Aufnahme sehr aufwändig und mögliche dynamische Effekte, die nur bei einem realen Bewegungsablauf auftreten, können nicht abgebildet werden.

Ist die Bewegung ausreichend langsam, wird beispielsweise die Kaubewegung ausreichend langsam ausgeführt, ist es auch möglich während eines einzigen Bewegungszyklus ausreichend magnetresonanztomographische Daten zu jeder Bewegungsphase zu erzeugen. Dies wird beispielsweise in " Real-Time Magnetic Resonance Imaging of Temporomandibular Joint Dynamics", S. Zhang et al., erschienen in The Open Medical Imaging Journal, 5, Seite 1-7, 2011, beschrieben.

Allerdings ist beispielsweise das langsame Ausführen einer Kaubewegung für einen Patienten sehr anspruchsvoll und muss trainiert oder unterstützt werden. Darüber hinaus können dynamische Effekte, die nur bei normaler Bewegungsgeschwindigkeit auftreten, mit diesem Verfahren nicht abgebildet werden.

Bei einer zyklischen Bewegung ist es weiterhin möglich, Datensätze über mehrere Zyklen der Bewegung zu generieren und anschließend den Datensätzen jeweils eine Phase der Bewegung zuzuordnen. So können alle der Phase entsprechenden Datensätze einem Bild der entsprechenden Bewegungsphase zugrunde gelegt werden.

Um die Zuordnung zu ermöglichen, ist es beispielsweise möglich, mittels einer Vorrichtung die Positionen der Bewegung zu protokollieren. Nachteilig ist jedoch der durch die zusätzliche Vorrichtung resultierende Aufwand.

Aus "Adaptive Averaging Applied to Dynamic Imaging of the Soft Palate" von A. D. Scott et al., erschienen in Magnetic Resonance in Medicine, Band 70, Seiten 865-874, September 2013, ist ein Verfahren zur Erzeugung von Aufnahmen von Bewegungen bekannt, welches Korrelationskoeffizienten zwischen einzelnen Echtzeitbildern bestimmt. Anhand der Korrelationskoeffizienten werden Echtzeitbilder derselben Bewegungsphase identifiziert und einem Gesamtbild dieser Bewegungsphase zugrundegelegt.

Das beschriebene Verfahren korreliert also Bilder mit einem geringen Signal-Rausch-Verhältnis (SNR), ohne dass hierzu eine zyklische Bewegung vorliegen müsste. Die Bilder mit einem hohen Korrelationskoeffizienten werden gemittelt, um das SNR zu erhöhen. Das Verfahren hat jedoch den Nachteil, dass durch die notwendige dichte Abtastung eines k-Raums, um artefaktfreie Bilder zu erhalten, die zeitliche und räumliche Auflösung begrenzt ist. Die räumliche Auflösung liegt beispielsweise zwischen 1,6 x 1,6 und 2,0 x 2,0 mm^2. Die zeitliche Auflösung kann beispielsweise aufgrund einer parallelen Bildgebung mit mehreren Spulen bei 50-111 ms liegen. Bei Messungen mit einer Spule läge die zeitliche Auflösung bei nur 150-300 ms. Die geforderte räumliche und zeitliche Auflösung für TMJ-Aufnahmen (Temporomandibular joint dysfunction) liegt bei maximal 0,75 x 0,75 mm^2 und 100 ms. Ein weiterer Nachteil ist, dass falsche oder zweideutige Maxima der paarweisen Korrelation zu verfälschten Ergebnissen führen können und das Verfahren hierdurch weniger robust ist.

Der Fachartikel J.P. Hulet, "Fast cardiac magnetic resonance imaging using radial trajectories, temporally constrained reconstruction, and graphics processing unit clusters", Dissertation, University of Utah/USA, 2013 offenbart die Verwendung einer Distanzmatrix, um Signale miteinander zu vergleichen. Dabei werden Funktionen an die sich in der Distanzmatrix ausbildenden Strukturen angepasst.

Die US 2011/0228987 A1 offenbart ein Verfahren zur Aufnahme eines bewegten Objekts. Die Bewegung des Objekts wird durch Vergleich der einzelnen Aufnahmen ermittelt. Dabei wird eine Ähnlichkeitsmatrix-Methode verwendet, die die Änderung bzw. die Distanzen der ähnlichen Strukturen in den einzelnen Aufnahmen ermittelt.

Der Fachartikel A. Anderson et al., • "Classification of spatially unaligned fMRI scans", NeuroImage 49 (2010), S. 2509-2519 offenbart ein Verfahren zur Klassifizierung von MRT-Aufnahmen, wobei Distanzmatrizen verwendet werden.

Der Fachartikel Wang et al., "On the Euclidean distance of images", IEEE Trans. Pattern Anal. Mach. Intell. 27 (2005), S. 1334-1339 offenbart eine mathematische Beschreibung von euklidischen Distanzmatrizen.

Die Aufgabe der vorliegenden Erfindung ist es, ein besonders einfaches und zuverlässiges Verfahren zur Erzeugung von Aufnahmen von einzelnen Bewegungsphasen einer zyklischen Bewegung mit einer hohen zeitlichen und räumlichen Auflösung bereitzustellen.

Offenbarung der Erfindung

Diese Aufgabe wird durch ein Verfahren zur Erzeugung magnetresonanztomographischer Bilder von mindestens einer Phase einer zyklischen Bewegung gelöst, welches die folgenden Verfahrensschritte umfasst:

Erzeugen von Rohdatensätzen einer zyklischen Bewegung während eines Aufnahmezeitraums T mit k-Raum-Teiltrajektorien; Rekonstruieren einer Serie von mindestens zwei Zwischenbildern, wobei jedes der Zwischenbilder aus jeweils mindestens einem Rohdatensatz zumindest für einen jeweiligen Ausschnitt (Region-of-Interest, ROI) der Rohdatensätze erstellt wird; Berechnen einer Distanzmatrix aus der Serie von Zwischenbildern, wobei jedes Matrixelement D dem Abstand eines Zwischenbilds der Serie zu sich selbst sowie zu einem weiteren Zwischenbild der Serie entspricht; Anpassen bzw. Fitten bzw. Annähern von Kurven bzw. Funktionen an sich in der Distanzmatrix ausbildende Strukturen und Rekonstruieren mindestens eines Bilds aus Rohdatensätzen, die eine Schnittmenge der gefitteten Kurven bzw. Funktionen mit der zu einem Zwischenbild gehörenden Einträge der Distanzmatrix entsprechen.

Die k-Raum-Teiltrajektorien können dabei radial oder nahezu radial sein.

Das Rekonstruieren der Zwischenbilder kann mit einer hohen Zeitauflösung erfolgen.

Die Distanzmatrix kann eine zweidimensionale, eine dreidimensionale oder eine mehrdimensionale Distanzmatrix sein. In einer zweidimensionalen Distanzmatrix können die Strukturen Linien sein.

Die Schnittmenge kann mehrere Schnittpunkte aufweisen.

Das Anpassen der Kurven bzw. Funktionen an Linien, die sich in der Distanzmatrix D ausgebildet haben kann beispielsweise über eine Maximierung der Kurvenintegrale erfolgen. Zur Maximierung der Kurvenintegrale kann beispielsweise ein Active-Contours-Verfahren oder auch ein anderes "Curve-Fitting"-Verfahren verwendet werden.

Alternativ dazu kann auch das sogenannte Brute-Force-Verfahren bzw. die sogenannte Exhaustionsmethode verwendet werden. Bei diesem Verfahren zu einer algorithmischen Lösung eines Problems werden alle potenziellen Lösungen durchprobiert, bis die richtige gefunden ist.

Das erfindungsgemäße Verfahren ermöglicht es, magnetresonanztomographische Bilder eines Objekts in einzelnen Phasen einer sich wiederholenden Bewegung zu erzeugen, ohne dass gesteigerte Anforderungen an die Ausführung der Bewegung oder die Geschwindigkeit der Datenakquisition gestellt werden müssen. Beispielsweise kann ein Kiefergelenk in den verschiedenen Phasen einer kontinuierlich in normalem Tempo ausgeführten Kau- oder einer sich wiederholenden Sprechbewegung abgebildet werden, indem die Bewegung über mehrere Zyklen vermessen wird.

Zur magnetresonanztomographischen Vermessung wird eine als Messsequenz bezeichnete zeitliche Abfolge von Kombinationen aus magnetischen Gradientenfeldern, Hochfrequenzimpulsen und Signalempfangszeiten verwendet, die während des Aufnahmezeitraums ein- oder mehrfach durchlaufen werden. Die Messsequenz gibt dabei beispielsweise die zeitlichen Verläufe der Frequenzen und Feldstärken der Gradientenfelder und Frequenzimpulse vor.

Aus der gewählten Messsequenz ergibt sich die k-Raum-Trajektorie, also die Abfolge der Messpunkte im k-Raum, wobei mit k-Raum ein zum Ortsraum Fouriertransformierter linearer Vektorraum eines zwei- oder dreidimensionalen komplexen Wellenvektors k bezeichnet wird. Entsprechend ist für den k-Raum auch die Bezeichnung Wellenvektorraum üblich.

Erfindungsgemäß wird eine aus radialen oder nahezu radialen k-Raum-Teiltrajektorien zusammengesetzte k-Raum-Trajektorie zur Abtastung des k-Raums verwendet. Als radiale k-Raum-Teiltrajektorie bezeichnet man einzelne durch einen Mittelpunkt des k-Raums verlaufende Linien mit Messpunkten. Diese können quasi als Kreisdurchmesser von außen durch den Mittelpunkt wieder nach außen verlaufen und auch Speichen, "Spokes" oder "center out-Linien" genannt werden. Ein Spezialfall sind "radial center-out" k-Raum-Teiltrajektorien, die lediglich vom Mittelpunkt nach außen verlaufen.

Ein Beispiel für nahezu radiale k-Raum-Trajektorien ist PROPELLER EPI (Magn Reson Med. 2005 Nov; 54(5):1232-40. "PROPELLER EPI: an MRI technique suitable for diffusion tensor imaging at high field strength with reduced geometric distortions", Wang et al.)

Die während des Aufnahmezeitraums bei der Abtastung des k-Raums gemessenen Rohdatensätze werden beispielsweise hintereinander in einer Speichereinheit abgelegt.

Ein Rohdatensatz kann das bei der Vermessung eines einzigen Punkts im k-Raum aufgezeichnete Signal sein. Ein Rohdatensatz kann auch die bei der Vermessung einer k-Raum-Teiltrajektorie aufgezeichneten Signale oder die Signale jeder beliebigen anderen Gruppierung aus Messpunkten im k-Raum enthalten. Häufig wird jeweils eine aus mehreren Messpunkten bestehende k-Raum-Teiltrajektorie bzw. die an den entsprechenden Messpunkten aufgenommenen Messdaten als ein Rohdatensatz abgespeichert.

Aus den Rohdatensätzen werden Zwischenbilder mit hoher Zeitauflösung rekonstruiert. Eine hohe Zeitauflösung kann durch eine niedrige Ortsauflösung oder durch eine hohe Ortsauflösung mit Abtastartefakten erreicht werden. Die Zeitauflösung wird beispielsweise erhöht, indem die Zahl der k-Raum-Teiltrajektorien bzw. Rohdatensätze pro Bild reduziert wird.

Durch eine Kombination mit paralleler Bildgebung kann die Zahl der k-Raum-Teiltrajektorien weiter reduziert werden.

Die Rekonstruktion kann für das gesamte örtliche Aufnahmefenster erfolgen. Durch eine Einschränkung der Rekonstruktion auf einen Ausschnitt, der von Interesse ist (Region-of-Interest, ROI), kann der Rechenaufwand reduziert werden. Weiterhin können hierdurch beispielsweise Einflüsse von den ein sich bewegendes Gelenk oder Objekt umgebenden Bereichen verringert und/oder vermieden und so die Qualität der zu erzeugenden Aufnahme verbessert werden. Die Gesamtheit dieser rekonstruierten Zwischenbilder wird als Serie von Zwischenbildern bezeichnet.

Aus der Serie von Zwischenbildern wird eine Distanzmatrix berechnet, wobei zu jedem Zwischenbild d'er Serie der Abstand zu jedem anderen Zwischenbild der Serie sowie zu sich selbst unter einer bestimmten Distanzmetrik bzw. Norm berechnet und als Matrixelement der Distanzmatrix abgelegt wird. Es kann beispielsweise eine euklidische Distanzmatrix mit den euklidischen Abständen der Zwischenbilder zueinander als Matrixelementen verwendet werden:

$$D = (d_{ij}); \quad d_{ij} = - \left\| z_i - z_j \right\|_2$$

Es kann aber auch jede andere Norm verwendet werden, die die Ähnlichkeit zwischen zwei Bildern beschreibt und damit als Ähnlichkeitsmaß dienen kann.

Der Fachartikel Wang et al., "On the Euclidean distance of images", IEEE Trans. Pattern Anal. Mach. Intell. 27 (2005), S. 1334-1339 offenbart eine mathematische Beschreibung von euklidischen Distanzmatrizen.

Die einem Vergleich jedes Zwischenbilds mit sich selbst entsprechenden, auf der Hauptdiagonale liegenden Matrixelemente weisen aufgrund der perfekten Gleichheit je nach Definition der Matrixelemente Minimal- oder Maximalwerte auf. In einer bildlichen Darstellung der Distanzmatrix ist daher deutlich eine Gerade entlang der Hauptdiagonalen zu erkennen. Parallel sowie orthogonal zu dieser Hauptdiagonalen bilden sich aufgrund des sich Wiederholens der Bewegung weitere ähnliche Strukturen bzw. Linien aus. Je gleichmäßiger sich die Bewegung wiederholt, desto ähnlicher sind die sich ausbildenden Linien der Hauptdiagonalen. Je zeitlich ungleichmäßiger sich die Bewegung wiederholt, desto mehr weichen die sich ausbildenden Linien von einem geraden Verlauf ab oder weisen Lücken auf.

Für jede Zeile der Distanzmatrix geben die Schnittpunkte der Linien mit der Zeile die geringsten Abstände, also die größte Übereinstimmung der zum Vergleich herangezogenen Zwischenbilder mit dem der Zeile zugrundeliegenden Zwischenbild wieder.

Um diese Schnittpunkte aufzufinden, werden die Linien jeweils mit einem Active-Contour-Verfahren, auch aktive Konturen oder Snakes genannt, durch eine Kurve bzw. einen Spline genähert bzw. gefittet. Hierfür wird jeweils von einer zur Hauptdiagonalen entsprechend parallel verschobenen Geraden als Startposition ausgegangen und durch eine Minimierung einer zugehörigen Energiefunktion ein eindimensionaler Spline, also ein eindimensionaler Polynomzug, an eine der Linien, also an deren Verlauf, angepasst.

Mittels der so gefundenen Kurven bzw. der Splines, werden die den Schnittpunkten der Linien mit einer Zeile der Distanzmatrix zugrundeliegenden Rohdatensätze ermittelt. Aus der so zu einer Zeile der Distanzmatrix ermittelten Untergruppe von Rohdatensätzen wird ein Bild rekonstruiert, welches genau eine Phase der Bewegung wiedergibt.

Vorteilhafterweise ist die Distanzmatrix eine euklidische Distanzmatrix. Die euklidische Distanzmatrix ist eine einfache Variante, eine Distanzmatrix umzusetzen.

Vorteilhafterweise kann der k-Raum mit einem Anordnungsschema abgetastet werden, das für jede zusammenhängende Untergruppe von k-Raum-Teiltrajektorien bzw. Spokes eine im k-Raum möglichst gleichverteilte Anordnung garantiert.

Das Abtasten kann beispielsweise nach einem aperiodischen Anordnungsschema erfolgen.

Für die Qualität der Bilder ist es wichtig, dass die k-Raum-Trajektorie, auch Sampling-Trajektorie genannt, so gewählt wird, dass jeweils die Winkel der einem Bild zugrunde zu legenden Untermenge von k-Raum-Teiltrajektorien im k-Raum möglichst gleichverteilt sind. Beim Vermessen einer zyklischen Bewegung besteht insbesondere die Gefahr, dass Schwebungen zwischen der Sampling-Trajektorie und der Bewegung auftreten, so dass Untergruppen von einzelnen einer Bewegungsphase zuordenbaren k-Raum-Teiltrajektorien keine möglichst gleichmäßige Abtastung des k-Raums und damit keine ausreichenden k-Raum-Informationen liefern können. Durch eine nicht-periodische Abtastung können Schwebungen der Abtastung des k-Raum mit der zyklischen Bewegung vermieden und die maximale Größe der Zwischenräume im k-Raum in den final einem Bild zugrunde zu legenden k-Raum-Teiltrajektorien minimiert werden. Dadurch wird die Qualität der Schicht- oder Volumenbilder verbessert.

Vorteilhafterweise schließen zeitlich aufeinanderfolgende radiale oder nahezu radiale k-Raum-Teiltrajektorien (kl... kx) einen Winkel ein, der dem Goldenen Winkel entspricht.

Der Goldene Winkel, also ein Winkel von 137,5° bzw. 222,5°, stellt eine einfache und sichere Möglichkeit dar, um Schwebungen der Abtastung des k-Raums mit der Zyklizität der Bewegung zu vermeiden und um die Größe der Zwischenräume in den final einem Bild zugrunde zu legenden k-Raum-Teiltrajektorien zu minimieren. Der Goldene Winkel garantiert für jede aufgrund der Bewegung zusammenhängende bzw. zusammengehörende Untermenge der k-Raum-Teiltrajektorien möglichst gleichverteilte Winkel.

Kurze Beschreibung der Zeichnungen

Das erfindungsgemäße Verfahren wird anhand der Zeichnung erläutert. Es zeigen:

Fig. 1    eine schematische Darstellung des Verfahrens,

Fig. 2    radiale k-Raum-Teiltrajektorien.

Ausführungsformen der Erfindung

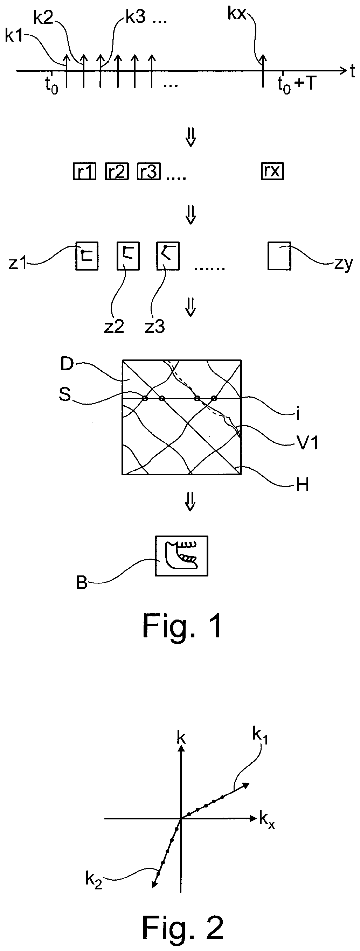

In Fig. 1 ist der Verlauf eines erfindungsgemäßen Verfahrens zumindest in Teilen anhand der Vermessung einer Kaubewegung schematisch dargestellt. Mittels eines aus dem Stand der Technik bekannten MRT-Systems wird während eines Zeitintervalls T ein Kiefergelenk eines Patienten vermessen, wobei der Patient während des Zeitintervalls T Kaubewegungen ausführt. Bei der Messung werden radiale k-Raum-Teiltrajektorien k1, k2, k3... kx abgetastet und die Messdaten in Form von Rohdatensätzen r1, r2, r3... rx erzeugt und beispielsweise nacheinander in einem Speicherbereich einer Speichereinheit abgelegt.

Der k-Raum wird beispielsweise gemäß dem Goldenen Winkel abgetastet, d.h., dass zwei zeitlich aufeinanderfolgende radiale k-Raum-Teiltrajektorien k1, k2, k3... kx immer einen Winkel einschließen, der dem Goldenen Winkel, also 137,5° bzw. 222,5°, entspricht, wie dies in Fig. 2 veranschaulicht ist.

Beispielhaft wird hier die Vermessung der Kaubewegung eines Kiefergelenks beschrieben. Es können aber auch eine sich wiederholende Sprechbewegung oder ein zyklisches Öffnen und Schließen des Munds oder auch anderer Gelenke in Bewegung vermessen werden, beispielsweise eines Kniegelenks. Es können aber auch andere sich bewegende Objekte vermessen werden, beispielsweise kann eine Atembewegung vermessen werden, wofür z.B. die Lunge-Leber-Kante als Region-of-Interest (ROI) ausgewählt werden kann.

Die Ausgestaltung des für die Vermessung verwendeten MRT-Systems richtet sich insbesondere nach dem zu vermessenden Gelenk oder Objekt bzw. nach dem zu vermessenden Bewegungsradius des Gelenks oder Objekts.

Je nach dem Zeitpunkt, zu dem ein Rohdatensatz r1, r2, r3... rx aufgenommen wurde, bildet er eine andere Phase der Bewegung ab, beispielsweise einen geschlossenen oder vollständig geöffneten Kiefer oder eine leicht geöffnete Kieferstellung während des Öffnens oder Schließens des Kiefers.

Da die Bewegung zyklisch ausgeführt wird, sich die verschiedenen Phasen also während des Zeitintervalls wiederholen, können für ein Bild einer bestimmten Phase der Bewegung mehrere zu unterschiedlichen Zeitpunkten aufgenommene Rohdatensätze herangezogen werden, die jeweils diese bestimmte Phase der Bewegung abbilden.

Um die Rohdatensätze r1, r2, r3... rx den verschiedenen Bewegungsphasen zuordnen zu können, werden aus den Rohdatensätzen Zwischenbilder z1, z2,... zy rekonstruiert, die zumindest einen die Bewegung abbildenden Ausschnitt, auch Region-of-Interest (ROI) genannt, mit einer hohen Zeitauflösung wiedergeben. Der Ausschnitt bzw. die ROI wird beispielsweise im Fall der Vermessung eines Kiefergelenks um den Kondylus gewählt.

Um eine ausreichende zeitliche Auflösung der Zwischenbilder z1, z2... zy zu erhalten, werden weniger radiale k-Raum-Teiltrajektorien bzw. Rohdatensätze pro Zwischenbild verwendet als durch die Auflösung vorgegeben ist, und dadurch Zwischenbilder mit einer niedrigen Ortsauflösung oder mit Aliasing-Artefakten rekonstruiert. Beispielsweise können aus einer jeweils geringen Anzahl von aufeinanderfolgenden Rohdatensätzen unterabgetastete Zwischenbilder rekonstruiert werden.

In einer Distanzmatrix D, z.B. einer euklidischen Distanzmatrix, werden die Abstände der Zwischenbilder z1, z2... zy zueinander, also jeweils ein Abstandsmaß, erfasst, wobei jede Zeile i der Distanzmatrix D den Abstand, z.B. den euklidischen Abstand, des Zwischenbilds zi zu den weiteren Zwischenbildern z1, z2, ... zy sowie zu sich selbst wiedergibt.

Aufgrund der perfekten Ähnlichkeit jedes Zwischenbilds z1, z2... zy zu sich selbst weist die Distanzmatrix D eine Hauptdiagonale H mit Minimalwerten auf. Weiterhin weist die Distanzmatrix D aufgrund der Zyklizität der Bewegung, also des mehrmaligen Auftretens bzw. Durchlaufens jeder der verschiedenen Bewegungsphasen, weitere, zur Hauptdiagonalen H parallel sowie senkrecht verlaufende Linien von Minimalwerten auf. Diese Linien verlaufen nur näherungsweise gerade, da die zyklische Bewegung, beispielsweise die Kaubewegung, zwar kontinuierlich, aber nicht unbedingt gleichförmig ausgeführt wird.

Anhand der Linien der Distanzmatrix können zu jedem Zwischenbild zi die Zwischenbilder zj mit der größten Ähnlichkeit gefunden werden, indem die Schnittpunkte S der Linien mit der Zeile i der Distanzmatrix D ermittelt werden. Hierzu wird jede Linie mittels eines Active-Contour-Verfahrens genähert bzw. gefittet.

Ein Active-Contour-Verfahren nähert einen Spline, also einen Polynomzug, an eine Kontur, indem eine entsprechend aufgestellte Energiefunktion minimiert wird. Der Spline wird im Folgenden auch als Kurve bzw. Funktion v1, v2... vz bezeichnet. Als Startwert für das Active-Contour-Verfahren kann von einer zur Hauptdiagonalen H parallel verschobenen Geraden ausgegangen werden.

Mittels der zu jeder Linie der Distanzmatrix D bestimmten bzw. gefitteten Kurven bzw. Funktionen v1, v2...vz (gepunktet dargestellt) werden die Schnittmengen S der Funktionen mit jeweils einer Zeile der Distanzmatrix D bestimmt. So kann zu jeder Zeile aus den den Matrixelementen der Schnittpunkte S zugrundeliegenden Rohdatensätzen r1, r2, r3... ry ein Bild B rekonstruiert werden, welches jeweils eine speziellen Bewegungsphase abbildet.

## Patentansprüche

1. Verfahren zur Erzeugung magnetresonanztomographischer Bilder (B) von mindestens einer Phase einer zyklischen Bewegung mit den Verfahrensschritten:

a. Erzeugen von Rohdatensätzen (rl... rx) einer zyklischen Bewegung während eines Aufnahmezeitraums (T) mit k-Raum-Teiltrajektorien (kl... kx) ;

b. Rekonstruieren einer Serie von mindestens zwei Zwischenbildern (z1... zy), wobei jedes der Zwischenbilder aus jeweils mindestens einem Rohdatensatz (rl... rx) zumindest für einen jeweiligen Ausschnitt, d.h. eine Region-of-Interest (ROI), der Rohdatensätze (rl... rx) erstellt wird;

c. Berechnen einer Distanzmatrix D aus der Serie von Zwischenbildern (z1... zy), wobei jedes Matrixelement D dem Abstand eines Zwischenbilds (z1... zy) der Serie zu sich selbst sowie mindestens einem weiteren Zwischenbild (z1... zy) der Serie entspricht;

d. Anpassen von Funktionen (v1... vz) an sich in der Distanzmatrix D ausbildende Strukturen und

e. Rekonstruieren mindestens eines Bildes Bi aus Rohdatensätzen (r1... rx), die einer Schnittmenge S der Funktionen (v1... vz) mit den zu einem Zwischenbild zi zugeordneten Einträgen der Distanzmatrix D entsprechen.

**2.** Verfahren nach Anspruch 1, **dadurch gekennzeichnet, dass** die Distanzmatrix (D) eine zweidimensionale euklidische Distanzmatrix ist.

**3.** Verfahren nach Anspruch 1 oder 2, **dadurch gekennzeichnet, dass** der k-Raum mit einem Anordnungsschema abgetastet wird, das für jede zusammenhängende Untergruppe von k-Raum-Teiltrajektorien (kl... kx) eine im k-Raum möglichst gleichverteilte Anordnung garantiert.

**4.** Verfahren nach einem der Ansprüche 1 bis 3, **dadurch gekennzeichnet, dass** zeitlich aufeinanderfolgende k-Raum-Teiltrajektorien (kl... kx) einen Winkel einschließen, der dem Goldenen Winkel entspricht.

## Claims

**1.** A method for generating magnet resonance tomography images (B) of at least one phase of a cyclic movement, having the method steps:

a. Generating raw data sets (r1... rx) of a cyclic movement during a recording period (T) with k-space sub-trajectories (k1... kx);

b. reconstructing a series of at least two intermediate images (z1... zy), each of the intermediate images being created from at least one raw data set (r1... rx) at least for a respective section, i.e. a region-of-interest (ROI) of the raw data sets (r1... rx);

c. calculating a distance matrix D from the series of intermediate images (z1... zy), each matrix element D corresponding to the distance between an intermediate image (z1... zy) of the series and itself and at least one further intermediate image (z1... zy) of the series;

d. adapting functions (v1... vz) to structures forming in the distance matrix D and

e. reconstructing at least one image Bi from raw data sets (r1... rx) which correspond to an intersection S of the functions (v1... vz) with the entries of the distance matrix D assigned to an intermediate image zi.

**2.** The method according to claim 1, **characterised in that** the distance matrix (D) is a two-dimensional Euclidean distance matrix.

**3.** The method according to claim 1 or 2, **characterised in that** the k-space is scanned using an arrangement scheme which guarantees an arrangement which is as evenly distributed as possible in the k-space, for each connected subgroup of k-space sub-trajectories (k1... kx).

**4.** The method according to any one of claims 1 to 3, **characterised in that** temporally successive k-space sub-trajectories (k1... kx) enclose an angle which corresponds to the golden angle.

## Revendications

**1.** Procédé pour générer des images par tomographie à résonance magnétique (B) d'au moins une phase d'un mouvement cyclique avec les étapes du procédé :

a. de génération d'enregistrements de données brutes (r1 ... rx) d'un mouvement cyclique pendant une période d'imagerie (T) avec des sous-trajectoires de l'espace k (k1 ... kx) ;

b. la reconstruction d'une série d'au moins deux images intermédiaires (z1 ... zy), chacune des images intermédiaires d'au moins un enregistrement de données brutes (r1 ... rx) respectif au moins pour une section respective, c'est-à-dire une région d'intérêt (ROI) des enregistrements de données brutes (r1 ... rx) étant créée ;

c. le calcul d'une matrice de distance D à partir de la série d'images intermédiaires (z1 ... zy), chaque élément de matrice D correspondant à la distance d'une image intermédiaire (z1 ... zy) de la série à elle-même et à au moins une autre image intermédiaire (z1 ... zy) de la série ;

d. l'adaptation des fonctions (v1 ... vz) aux structures qui se forment dans la matrice de distance D et

e. la reconstruction d'au moins une image Bi à partir d'enregistrements de données brutes (r1 ... rx) qui correspondent à une intersection S des fonctions (v1 ... vz) avec les entrées de la matrice de distance D affectées à une image intermédiaire zi.

2. Procédé selon la revendication 1, **caractérisé en ce que** la matrice de distance (D) est une matrice de distance euclidienne bidimensionnelle.

3. Procédé selon la revendication 1 ou 2, **caractérisé en ce que** l'espace k est balayé en utilisant un schéma d'agencement qui garantit un agencement qui est réparti aussi uniformément que possible dans l'espace k pour chaque sous-groupe connecté de sous-trajectoires d'espace k (k1 ... kx).

4. Procédé selon l'une quelconque des revendications 1 à 3, **caractérisé en ce que** des sous-trajectoires temporellement successives de l'espace k (k1 ... kx) renferment un angle qui correspond à l'angle d'or.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110228987 A1

**In der Beschreibung aufgeführte Nicht-Patentliteratur**

- **S. ZHANG et al.** Real-Time Magnetic Resonance Imaging of Temporomandibular Joint Dynamics. *The Open Medical Imaging Journal,* 2011, vol. 5, 1-7
- **A. D. SCOTT et al.** Adaptive Averaging Applied to Dynamic Imaging of the Soft Palate. *Magnetic Resonance in Medicine,* September 2013, vol. 70, 865-874
- Fast cardiac magnetic resonance imaging using radial trajectories, temporally constrained reconstruction, and graphics processing unit clusters. **J.P. HULET.** Dissertation. University of Utah, 2013
- **A. ANDERSON et al.** Classification of spatially unaligned fMRI scans. *NeuroImage,* 2010, vol. 49, 2509-2519
- **WANG et al.** On the Euclidean distance of images. *IEEE Trans. Pattern Anal. Mach. Intell.,* 2005, vol. 27, 1334-1339
- **WANG.** PROPELLER EPI: an MRI technique suitable for diffusion tensor imaging at high field strength with reduced geometric distortions. *Magn Reson Med.,* November 2005, vol. 54 (5), 1232-40